# EUROPÄISCHE PATENTANMELDUNG

(11) **EP 2 982 365 A2**
(43) Veröffentlichungstag der Anmeldung: **10.02.2016**
(21) Anmeldenummer: 15181985.1
(22) Anmeldetag: 29.05.2013
(51) Int. Cl.: A61K 8/49, A61K 8/67, A61Q 19/08

(54) **KOSMETISCHE UND DERMATOLOGISCHE ZUBEREITUNG ENTHALTEND EINE ODER MEHRERE SUB-STANZ(EN), DIE DAS GEN / PROTEIN FÜR DEN REZEPTOR ENDO180 MODULIEREN**

(30) Priorität: 20.06.2012 DE 102012210384
(62) Teilanmeldung aus: 13726183.0
(71) Anmelder: Beiersdorf AG, 20253 Hamburg (DE)
(72) Erfinder: WEISE, Julia, 22529 Hamburg (DE); TANG, Stefanie, 20251 Hamburg (DE); VON WEDEL-PARLOW, Magdalena, 20251 Hamburg (DE)

(57) **Zusammenfassung**

Verwendung von einer oder mehreren Substanz(en), gewählt aus folgender Gruppe mit anellierten / kondensierten bizyklischen Verbindungen
Coffein (58-08-2), Tocopherol (10191-41-0) und (-)-Catechin (18829-70-4)
zur Stimulierung der Expression von Endo180 zur Aktivierung des Kollagen-Stoffwechsels in der Altershaut und zur Anregung der Altershaut, defektes Kollagen zu beseitigen.

## Beschreibung

Die Erfindung umfasst kosmetische und dermatologische Zubereitungen enthaltend eine oder mehrere Substanz(en) die das Gen / Protein für den Rezeptor Endo180 modulieren, insbesondere solche Zubereitungen zur Verwendung auf der Haut eines Menschen zur Reparatur und Regeneration der menschlichen Haut, insbesondere zur verbesserten Hautkonturierung bzw. gegen alterungsbedingte Hautfaltenbildung.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen:
a) Trockenheit, Rauhigkeit und Ausbildung von Trockenheitsfältchen,
b) Juckreiz
c) dünnere Haut
d) verringerte epidermale Zellteilungsrate
e) verringerter Energiestoffwechsel und
f) verminderte Rückfettung durch Talgdrüsen (z.B. nach Waschen).

Exogene Faktoren, wie UV-Licht und chemische Noxen, können kumulativ wirksam sein und z.B. die intrinsischen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
g) Sichtbare Gefäßerweiterungen (Teleangiektasien, Cuperosis);
h) Schlaffheit und Ausbildung von Falten;
i) lokale Hyper-, Hypo- und Fehlpigmentierungen (z.B. Altersflecken) und
j) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut (intrinsische und extrinsische Hautalterung), sowie der unter a), c), e), h) und j) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Bei der Alterung des menschlichen Körpers kommt es in vielen Organen zu einer Verlangsamung von Stoffwechselaktivitäten. So bauen bspw. im Knochen spezielle Zellen - die Osteoklasten - Knochenmasse ab und ihre Gegenspieler - die Osteoblasten - Knochenmasse auf. Es dauert im Durchschnitt 200 Tage bis das gesamte Knochenkalzium eines Erwachsenen einmal komplett ausgetauscht wurde. Allerdings lässt der Knochenstoffwechsel mit zunehmendem Alter nach: Ab dem 35. Lebensjahr verliert der Mensch durchschnittlich 1,5 % Knochenmasse pro Jahr. Ähnlich verhält es sich in der Haut. Die Zellen der oberen Hautschicht (Epidermis) erneuern sich durch Differenzierung der Keratinozyten zu Korneozyten und Abschilfern innerhalb von ca. 28 Tagen bei jungen Menschen. Im Alter dauert der Prozess bis zu 50 Tage.

Das Hauptbindegewebsprotein Kollagen, welches der Haut Spannkraft und Zugfestigkeit verleiht, unterliegt auch einem Stoffwechsel, angefangen bei der Kollagen-Synthese über die - Fibrillogenese bishin zur -Degradation und -Endocytose. Der Kollagen-Umsatz ist ein relativ langsamer Prozess, denn es sind Halbwertzeiten für Kollagen von 10-15 Jahren beschrieben (Verzijl et al, 2000).

Kollagen ist das prominenteste Protein in der menschlichen Haut und macht ca. 85 % der Gesamtproteinmenge aus. Damit haben die Kollagenfasern einen entscheidenden Einfluss auf das äußere Erscheinungsbild der Haut.

Der Kollagenumsatz in der Haut hängt von der Balance zwischen der Neubildung und dem Abbau des Kollagens ab. Durch die intrinsische und insbesondere extrinsische Hautalterung kommt es zu einer verminderten Kollagen-Synthese und einem verstärkten Abbau durch Kollagen degradierende Enzyme wie die Matrixmetalloproteasen (MMPs). Dadurch wird die Balance gestört, und es kommt zu einem Verlust an dermalem Kollagen, was zur Faltenbildung und nachlassenden Spannkraft der Altershaut beiträgt. Im Alter wird ein jährlicher Verlust von ca. 1 % der Collagenmenge in der Haut beschrieben (Shuster et al, 1975).

Die Integrität des Kollagens und anderer Bestandteile der Extrazellulären Matrix (ECM) hat einen entscheidenden Einfluss auf die Wechselwirkung der Kollagenfasern mit den Fibroblasten, die die verschiedenen Komponenten der ECM synthetisieren können. Fibroblasten besitzen verschiedene Rezeptoren für Kollagen, und zwar insbesondere Integrine, Discoidin Domain Rezeptoren (DDRs) und Mitglieder der Mannose Rezeptorfamilie wie Endo180 (Leitinger und Hohenester, 2007). Die Rezeptoren DDR2 und Endo180 wurden in Hautfibroblasten der Maus in Zusammenhang mit Wundheilungsvorgängen und Remodellierungsvorgängen nachgewiesen (Olaso et al, 2002, Madsen et al, 2007). DDR2 ist eine Rezeptor-Tyrosinkinase, die u.a. für die Fibroblasten Proliferation aber auch für die Kollagen-Fibrillogenese von Bedeutung ist. Endo180 hingegen ist für das endocytotische Entfernen von sowohl intaktem Kollagen als auch Kollagen-Bruchstücken wichtig. Nach der Rezeptor-vermittelten Kollagen-Endocytose fusionieren die entstandenen Vesikel mit Lysosomen, und die Kollagenfragmente werden anschließend durch Cathepsine abgebaut (Rünger et al, 2007). Es gibt Hinweise darauf, dass denaturierte Kollagene besser als native aufgenommen werden. Bei Fibroblasten, die auf denaturiertem Kollagen *versus* nativem Kollagen kultiviert wurden, zeigen erstere eine erhöhte Kollagen-Aufnahme und COL1A1 Expression (Abraham et al, 2007).

Während die Rolle verschiedener Integrine auf Fibroblasten (α₂β₁, α₁₁β₁, α₁β₁) für den Matrix-Abbau und deren Erneuerung schon relativ gut untersucht ist (White et al, 2004), ist es noch weitestgehend unbekannt, inwieweit die nicht-Integrin-Rezeptoren einen Anteil am Kollagenumsatz in gesunder Haut haben.

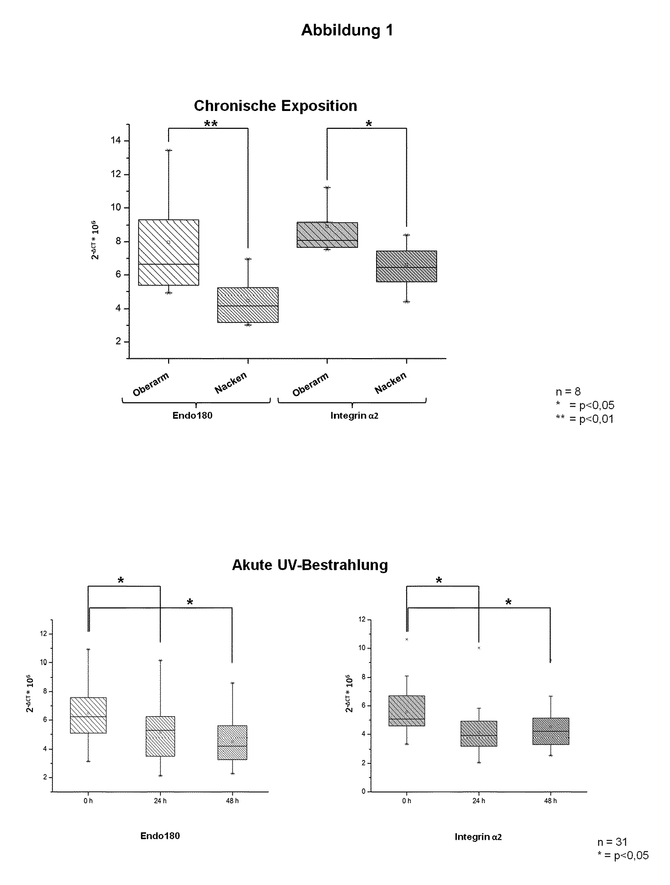

Endo180 sowie Integrin α₂ß₁ sind Rezeptoren auf der Oberfläche von mesenchymalen Zellen wie dermalen Fibroblasten und für die Aufnahme von Kollagen verantwortlich. Es konnte gezeigt werden, dass Endo180 und Integrin α₂ in photogealterter Haut bzw. nach akutem UV Stress *in vivo* herunter reguliert werden (Abb. 1).

### Abb. 1: Genexpressionsanalyse von Endo180 und Integrin α₂ in photogealterter Haut bzw. nach akutem UV Stress in vivo.

A) Genexpressionsanalyse von Endo180 und Integrin α₂ in chronisch sonnenexponierter (Nacken) *versus* sonnengeschützter Haut. Probanden mit einer klinisch diagnostizierten solaren Elastose im Nacken wurden Vollhautbiopsien aus einem sonnenexponierten Areal (Nacken) und einem sonnengeschützten Areal (innerer Oberarm) entnommen. Die mRNA-Werte wurden auf die 18S rRNA-Werte normiert. n=8. B) Genexpressionsanalyse von Endo180 und Integrin α₂ nach akutem UV Stress (2 *Minimal Erythemal Dose* (MED)) *in vivo.* Nach 24h und 48h wurden Vollhautstanzen (Gesäß) für die Analyse entnommen. Als Kontrolle dienten Stanzbiopsien aus unbestrahlten Arealen. Die mRNA-Werte wurden auf die 18S rRNA-Werte normiert. Dargestellt ist die Expression der Rezeptoren zu den jeweiligen Zeitpunkten nach Bestrahlung. n=31

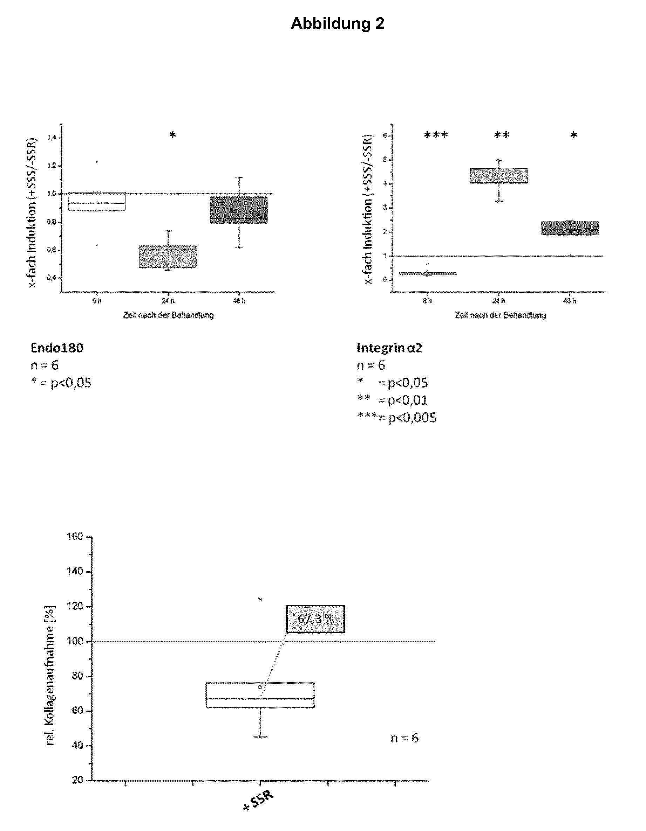

*In vitro* konnten diese Ergebnisse mir SSR (*solar simulated radiation*) bestrahlten humanen primären Fibroblasten nachvollzogen werden. Auch hier führt der UV-Stress zu einer verminderten Genexpression von Endo180 und Integrin α₂ sowie einer verringerten Kollagenaufnahme durch die Zellen (Abb. 2).

### Abb. 2: Genexpressionsanalyse von Endo180 und Integrin α₂ sowie Kollagen-Endocytose nach akutem UV-Stress in Fibroblasten in vitro.

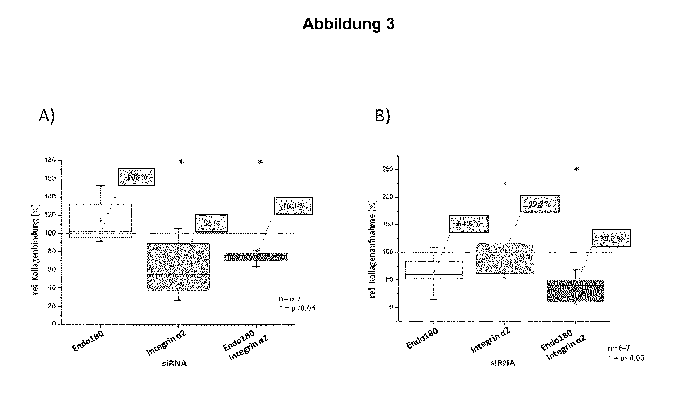

A) Expressionsprofil der Kollagenrezeptoren Endo180 und Integrin α₂ nach SSR-Bestrahlung (135 mJ/cm²) nach 6h, 24h und 48h. Die Darstellung ist bezogen auf die unbehandelte Kontrolle. Die mRNA-Werte wurden auf die 18S rRNA-Werte normiert. B) Analyse der Kollagen-Internalisierung mittels Durchflusszytometrie. Einen Tag nach SSR-Bestrahlung (135 mJ/cm²) wurden die Zellen 24h mit Kollagen-Fluoreszein [25 µg/ml] inkubiert und der Anteil an Zellen bestimmt, die Kollagen-Fluoreszein aufgenommen haben. Als Kontrolle dienten unbestrahlte Zellen. Es zeigte sich, dass die Kollagenaufnahme nach SSR-Bestrahlung um ca. 30 % reduziert war. n=6 *Knockdown* Experimente in Fibroblasten zeigten, dass Endo180 essentiell für die Kollagenaufnahme ist, während Integrin α₂ wichtig für die initiale Bindung von Kollagen ist und somit dessen Aufnahme erleichtert (Abb. 3).

### Abb. 3: Kollagen-Adhäsion und -Endocytose nach Endo180 und/oder Integrin α₂ knockdown.

Analyse der Kollagen-Fluoreszein-Bindung bzw. -Aufnahme mittels Durchflusszytometrie. Sechs Tage nach siRNA-Transfektion wurden die *knockdown* Zellen trypsinisiert und neu ausgesät. Nach 5 Stunden wurde Kollagen-Fluoreszein [25 µg/ml] zu den Fibroblasten gegeben. Nach einer 24-stündigen Inkubation wurde der Anteil an Zellen bestimmt, die Kollagen-Fluoreszein oberflächlich gebunden bzw. aufgenommen haben. Als Kontrolle dienten Fibroblasten, die mit einem *Non-Targeting Pool* transfiziert wurden (= 100 %). A) Es zeigte sich, dass die Kollagenbindung in Integrin α₂ *knockdown* Fibroblasten um 45 % reduziert war. In Endo180-/Integrin α₂ Doppel*-knockdown* Fibroblasten war die Kollagenbindung um ca. 25 % reduziert. Der Endo180 *knockdown* alleine zeigte im Mittel keinen Effekt. B) Es zeigte sich, dass die Kollagenaufnahme in Endo180 *knockdown* Fibroblasten um ca. 35 % reduziert war. In Endo180-/Integrin α₂ *knockdown* Fibroblasten war die Kollagenaufnahme um ca. 60 % reduziert. Der Integrin α₂ *knockdown* alleine zeigte im Mittel keinen Effekt. n_{Endo180}=6, n_{ITGA2}, _{Endo180/ITGA2}=7

Damit wird folgendes abgeleitet:
▪ Durch UV werden MMPs induziert
▪ Kollagen wird degradiert
▪ Endo180 und Integrin α₂ werden reprimiert
▪ Der Fibroblast wird perizellulär von degradiertem Kollagen umgeben
▪ Verlust der Zell-Matrix-Kontakte

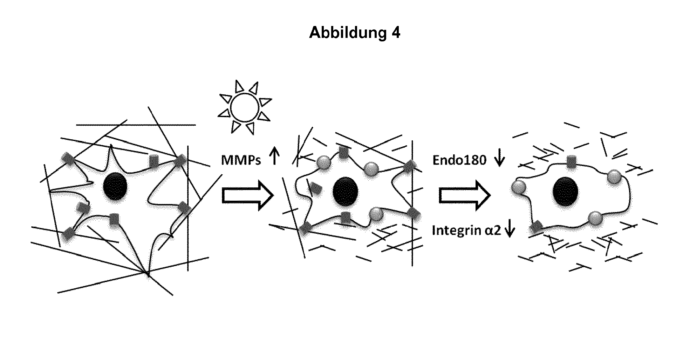

Auf einer Posterpräsentation (Abbildung 4) beim "Annual Meeting of the Society for Investigative Dermatology" 2012 wurde eine Stimulation der Endo180 Expression in gealterter Haut von der Arbeitsgruppe um Fisher gezeigt. Hier wird jedoch nicht darauf eingegangen, ob es sich um chronologisch oder photogealterte Haut handelt.

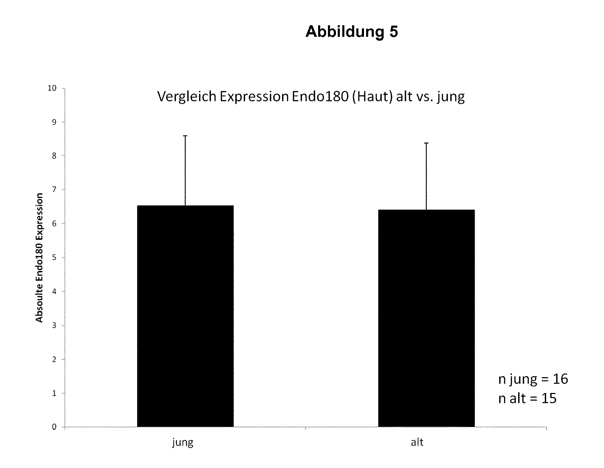

Die der vorliegenden Erfindung zugrundeliegenden Daten zeigen dagegen eine signifikante Inhibition der der Endo180 Expression in photogealterter Haut, während wir in chronologisch gealterter Haut keine Regulation der Endo180 Expression sehen (Abb. 5).

### Abb. 5: Genexpressionsanalyse von Endo180 in junger vs. intrinsisch gealterter Haut in vivo.

Genexpressionsanalyse von Endo180 in der Haut (Gesäß) von jungen (21-31 Jahre) vs. alten (61-70 Jahre) Spendern. Die mRNA-Werte wurden auf die 18S rRNA-Werte normiert. n_{jung}=16, nₐₗₜ=15

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten.

In der vorliegenden Erfindung konnte gezeigt werden, dass der Rezeptor Endo180 verantwortlich für die Aufnahme von Kollagenfragmenten durch Fibroblasten ist. Seine Expression nimmt in photogealterter Haut ab. Es wäre wünschenswert, die Expression von Endo180 in der Altershaut als *anti-aging* Ansatz zu stimulieren.

Überraschenderweise konnte in einem *in vitro* Modell einerseits festgestellt werden, dass eine oder mehrere Substanz(en), gewählt aus folgender Gruppe mit anellierten / kondensierten bizyklischen Verbindungen
Coffein (58-08-2), Tocopherol (10191-41-0) und (-)-Catechin (18829-70-4)
die Expression von Endo180 stimulieren. Diese werden im Rahmen der Offenbarung dieser Erfindung auch mit Begriffen wie "die erfindungsgemäß eingesetzten Wirkstoffe" bezeichnet werden.

| | | |
|---|---|---|
| 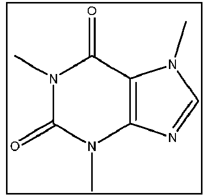 | Substanz | Coffein |
| | IUPAC | 1,3,7-Trimethyl-1H-purin-2,6(3H,7H)-dion |
| | CAS-Nr. | 58-08-2 |
| | | |
| 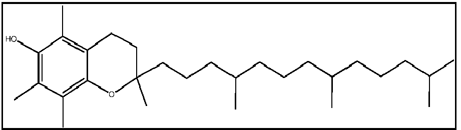 | Substanz | Tocopherol |
| | IUPAC | 2,5,7,8-Tetramethyl-2-(4,8,12-trimethyltridecyl)chroman-6-ol |
| | CAS-Nr. | 10191-41-0 |
| | | |
| 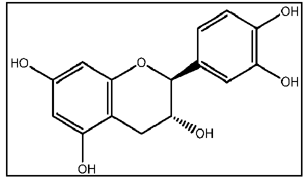 | Substanz | (-)-Catechin |
| | IUPAC | (2S,3R)-2-(3,4-Dihydroxyphenyl)chroman-3,5,7-triol |
| | CAS-Nr. | 18829-70-4 |

Der Einsatz dieser stimulierenden Substanzen in kosmetischen und dermatologischen Zubereitungen kann zur Verminderung des Erscheinungsbilds gealterter Haut (z.B. Faltenbildung, Verlust der Hautfestigkeit) führen.

Erfindungsgemäß ist daher die Verwendung von einer oder mehreren Substanz(en), gewählt aus folgender Gruppe mit anellierten / kondensierten bizyklischen Verbindungen
Coffein (58-08-2), Tocopherol (10191-41-0) und (-)-Catechin (18829-70-4)
geeignet zur Stimulierung der Expression von Endo180.

Insbesondere ist die Verwendung von einer oder mehreren Substanz(en), gewählt aus folgender Gruppe mit anellierten / kondensierten bizyklischen Verbindungen
Coffein (58-08-2), Tocopherol (10191-41-0) und (-)-Catechin (18829-70-4)
zur Glättung der Haut und/oder der Hautkonturen
eine vorteilhafte Ausführungsart der vorliegenden Erfindung.

Besonders vorteilhafte erfindungsgemäß eingesetzte Wirkstoffen sind Tocopherol (10191-41-0) und (-)-Catechin (18829-70-4).

**Tab. 1:**

| Humane dermale Fibroblasten wurden für drei Tage mit der jeweiligen Substanz [10 µM] inkubiert. Als Kontrolle dienten unbehandelte Zellen. Anschließend wurde die Endo180 Expression mittels Antikörper-Nachweis im Cell Western gemessen und quantifiziert. Zur Normierung der Werte wurden die Zellen mit DRAQ5/Sapphire700 gefärbt. Die Kontrolle wird als 100 % dargestellt. Leicht Endo180 stimulierende Substanzen zeigen einen Wert von 105 - 109 %, stimulierende Substanzen 110-114 % und stark stimulierende Substanzen ≥115%. | | |
|---|---|---|
| **Substanz** | **CAS** | **Ergebnis [%]** |
| Coffein | 58-08-2 | **107** |
| Tocopherol | 10191-41-0 | **111** |
| (-)-Catechin | 18829-70-4 | **119** |

Die Verwendung erfindungsgemäßer Zubereitungen mit einem kosmetisch wirksamen Gehalt an einem oder mehreren erfindungsgemäß eingesetzten Wirkstoffen - z. B. in Form der genannten Beispiele - führt in überraschender Weise zu einer erheblichen Verbesserung des Aussehens der gealterten Haut.

Es war überraschend, dass durch Anwenden von Zubereitungen gemäß der Erfindung die Hautfestigkeit verbessert wird.

Bevorzugt enthalten kosmetische oder dermatologische Zubereitungen gemäß der Erfindung 0,00001 bis 50 Gew.-%, bevorzugt 0,001 bis 20 Gew.-%, an einem oder mehreren erfindungsgemäß eingesetzten Wirkstoffen, bezogen auf das Gesamtgewicht der Zubereitung.

Die erfindungsgemäßen kosmetischen oder dermatologischen Zubereitungen können wie üblich zusammengesetzt sein und zur Behandlung, der Pflege und der Reinigung der Haut dienen. Sie enthalten bevorzugt 0,002 - 10,0 Gew.-%, besonders bevorzugt 0,002 - 5,0 Gew.-%, ganz besonders bevorzugt 0,005 - 3,0 Gew.-%, bezogen auf das Gesamtgewicht der Zubereitungen, an einem oder mehreren erfindungsgemäß eingesetzten Wirkstoffen.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Die wässrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs).

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Rezepturbeispiel** | **1** | **2** | **3** | **4** |
|---|---|---|---|---|
| **INCI - Bezeichnung** | **Gew.-%** | **Gew.-%** | **Gew.-%** | **Gew.-%** |
| Stearinsäure | 2,5 | 2,0 | 2,0 | 2,5 |
| Glyceryl Stearat | 1,0 | 1,0 | 1,0 | 1,0 |
| C12-15 Alkyl Benzoat | 3,0 | 5,00 | 3,0 | 2,0 |
| Caprylsäure/Caprinsäure Triglyceride | 2,5 | 2,50 | 2,0 | 2,5 |
| Isopropyl Palmitat | 2,0 | - | - | 2,0 |
| CetylStearylalkohol | 3,0 | - | 2,0 | 3,0 |
| Cetyl Alkohol | - | 2,00 | - | - |
| Stearyl Alkohol | - | 2,00 | 1,0 | - |
| Cyclomethicon | 1,0 | 1,0 | 0,5 | - |
| Dicaprylyl Carbonate | 2,0 | 2,00 | 2,00 | 2,0 |
| Dimethicon | 1,0 | - | 0,5 | 1,0 |
| Glycerin | 5, | 7,0 | 5,0 | 9,0 |
| Methylparaben | 0,2 | - | - | - |
| Phenoxyethanol | 0,4 | 0,50 | 0,5 | 0,4 |
| Propylparaben | 0,1 | - | - | 0,1 |
| 1,2-Hexandiol | - | - | 0,1 | 0,1 |
| Ethylhexylglycerin | - | - | 0,2 | - |
| Methylisothiazolinon | - | 0,05 | - | - |
| Butylenglykol | - | - | 2,0 | - |
| Carbomer | 0,15 | 0,10 | 0,15 | 0,1 |
| Carrageenan | 0,10 | - | 0,10 | - |
| Xanthan Gummi | - | - | 0,10 | - |
| Acrylates/C10-30 Alkyl Acrylate Crosspolymer | - | 0,10 | - | 0,1 |
| Trinatrium EDTA | 0,20 | 0,20 | 0,2 | 0,2 |
| Tapioka Stärke | 1,5 | 1,0 | - | |
| Nylon-12 | - | 0,2 | - | 0,5 |
| Polymethylsissesquioxane | - | 1,0 | 1,0 | - |
| Aluminum Stärke Octenylsuccinat | - | - | 1,0 | - |
| Distärkephosphat | 1,0 | - | - | 1,0 |
| Butyl Methoxydibenzoylmethane | 1,00 | 2,00 | 1,00 | 1,00 |
| Phenylbenzimidazole Sulfonic Acid | - | 1,00 | 2,00 | - |
| Octocrylene | - | 2,00 | 1,00 | - |
| Ethylhexyl Salicylate | 1,00 | - | - | 1,00 |
| Natriumhydroxid | q.s. | q.s. | q.s. | q.s. |
| Coffein | 0,1 | - | - | 0,05 |
| Tocopherol | 0,005 | 0,01 | - | 0,05 |
| (-)-Catechin | - | 0,005 | 0,01 | - |
| Parfüm | 0,3 | 0,2 | 0,2 | 0,2 |
| Natriumhydroxid | q.s. | q.s. | q.s. | q.s. |
| Wasser | ad 100 | ad 100 | ad 100 | ad 100 |

| **Rezepturbeispiel** | **5** | **6** | **7** | **8** |
|---|---|---|---|---|
| **INCI - Bezeichnung** | **Gew.-%** | **Gew.-%** | **Gew.-%** | **Gew.-%** |
| Glyceryl Stearate Citrat | 2,0 | 1,5 | 2,0 | 2,0 |
| Behenyl Alkohol | 1,5 | 1,0 | 1,0 | 1,0 |
| C12-15 Alkyl Benzoat | 2,0 | 2,5 | 2,0 | 2,5 |
| Caprylsäure/Caprinsäure Triglyceride | 2,0 | 2,0 | 2,5 | 2,5 |
| Cetyl Alkohol | 2,0 | 2,0 | - | 2,0 |
| Cetylstearylalkohol | - | - | 2,0 | - |
| Cyclomethicon | 1,0 | 1,0 | 2,0 | 2,0 |
| Dicaprylyl Carbonat | - | 2,0 | 2,5 | 2,5 |
| Paraffinum Liquidum | - | - | 0,5 | - |
| Octyldodecanol | - | 2,0 | - | - |
| Dimethicon | 0,5 | 1,00 | 1,00 | - |
| Glycerin | 3,00 | 5,00 | 7,00 | 9,00 |
| Methylparaben | 0,20 | 0,15 | - | - |
| Phenoxyethanol | 0,40 | 0,60 | 0,5 | 0,50 |
| Propylparaben | 0,10 | - | - | - |
| Methylisothiazolinon | - | - | 0,05 | - |
| Piroctone Olamine | - | - | - | 0,15 |
| Glyceryl Caprylate | - | - | - | 0,2 |
| Carbomer | 0,20 | - | 0,15 | 0,15 |
| Natrium Polyacrylate | - | 0,4 | - | - |
| Xanthan Gummi | 0,10 | - | 0,10 | - |
| Acrylates/C10-30 Alkyl Acrylate Crosspolymer | - | 0,1 | - | 0,1 |
| Tapioka Stärke | 0,50 | - | 0,50 | - |
| Nylon-12 | 1,0 | - | - | 1,0 |
| Polymethylsissesquioxane | - | 1,0 | 1,0 | - |
| Aluminum Stärke Octenylsuccinat | - | 1,0 | - | 1,00 |
| Glycyrrhiza Inflata Root Extract | 0,03 | 0,05 | 0,05 | 0,03 |
| Titandioxid | - | 1,0 | - | - |
| Octocrylene | - | 1,0 | - | 2,0 |
| Bis-Ethylhexyloxyphenol Methoxyphenyl Triazines | - | 1,0 | 1,0 | - |
| 2-Ethylhexyl Methoxycinnamate | - | 1,0 | 2,0 | 2,0 |
| Homosalat (3,3,5-Trimethyl-cyclohexylsalicylat) | - | - | 1,0 | 1,0 |
| Natriumhydroxid | q.s. | q.s. | q.s. | q.s. |
| Coffein | 0,1 | - | q.s. | q.s. |
| Tocopherol | - | 0,005 | - | - |
| (-)-Catechin | - | - | 0,01 | - |
| Parfüm | 0,1 | 0,2 | 0,3 | 0,2 |
| Wasser | ad 100 | ad 100 | ad 100 | ad 100 |

| **Rezepturbeispiele** | **9** | **10** | **11** | **12** |
|---|---|---|---|---|
| **INCI - Bezeichnung** | **Gew.-%** | **Gew.-%** | **Gew.-%** | **Gew.-%** |
| Polyglyceryl-3 Methylglucose Distearate | 2,00 | 2,50 | 2,50 | 2,50 |
| Sorbitan Stearate | 1,50 | 3,00 | 1,50 | 3,00 |
| C12-15 Alkyl Benzoate | 2,50 | 2,50 | 2,50 | 2,50 |
| Caprylsäure/Caprinsäure Triglyceride | 2,50 | 2,50 | 2,50 | 2,50 |
| Stearyl Alcohol | 1,00 | 1,50 | 1,00 | 1,50 |
| Cyclomethicone | 3,00 | 1,00 | 2,00 | 1,00 |
| Isopropyl Myristat | - | 2,50 | 2,0 | 2,50 |
| Isopropyl Palmitat | 2,0 | - | 1,0 | - |
| Dimethicone | - | 1,00 | - | 1,00 |
| Glycerin | 5,00 | 7,50 | 3,00 | 7,50 |
| Butyrospermum Parkii Butter | 2,0 | - | - | - |
| Methylparaben | 0,20 | 0,20 | - | 0,1 |
| Phenoxyethanol | 0,40 | 0,40 | 0,40 | 0,40 |
| Propylparaben | 0,10 | - | - | - |
| Benzethonium chlorid | - | - | 0,1 | - |
| Caprylyl Glycol | - | 0,2 | - | - |
| Ethylhexylglycerin | - | 0,2 | - | 0,2 |
| Carbomer | 0,15 | 0,10 | 0,15 | 0,10 |
| Ammonium Acryloyldimethyltaurate/VP Copolymer | - | 0,2 | - | 0,2 |
| Carrageenan | 0,1 | - | 0,15 | - |
| Trisodium EDTA | - | 1,00 | - | 1,00 |
| Tapioka Stärke | - | 1,00 | 1,0 | - |
| Distärke Phosphat | - | 1,00 | - | 1,0 |
| Acrylonitrile-methacrylonitrile-methyl-methacrylate Copolymer + Isopentane + Magnesium Hydroxide | - | - | 1,0 | 1,0 |
| Diethylamino Hydroxybenzoyl Hexyl Benzoate | - | - | 1,0 | - |
| Ethylhexyl Methoxycinnamate | - | 1,00 | - | 2,00 |
| Butyl Methoxydibenzoylmethane | - | 2,00 | - | 2,00 |
| Octocrylen | - | 1,00 | 2,0 | 1,00 |
| Titandioxid | - | - | 1,0 | - |
| Natrium Hydroxid | q.s. | q.s. | q.s. | q.s. |
| Ubiquinone | 0,1 | - | - | - |
| Natrium Metabisulfite | - | 0,15 | - | - |
| BHT (tert-Butylhydroxytoluol) | - | - | 0,05 | - |
| Coffein | 0,05 | - | - | - |
| Tocopherol | - | 0,05 | - | - |
| (-)-Catechin | - | - | 0,05 | - |
| Parfüm | q.s. | q.s. | q.s. | q.s. |
| Wasser | ad 100 | ad 100 | ad 100 | ad 100 |

| **Rezepturbeispiele** | **13** | **14** | **15** | **16** |
|---|---|---|---|---|
| **INCI - Bezeichnung** | **Gew.-%** | **Gew.-%** | **Gew.-%** | **Gew.-%** |
| PEG-40 Stearate | 0,80 | 1,00 | 1,00 | 1,00 |
| Glyceryl Stearate | 2,50 | 3,00 | 3,00 | 3,00 |
| C12-15 Alkyl Benzoate | 2,00 | 2,50 | 2,00 | 2,00 |
| Caprylsäure/Caprinsäure Triglyceride | 2,00 | 2,50 | 2,50 | 2,00 |
| Cetylstearylalkohol | 3,00 | 3,00 | 3,00 | 3,00 |
| Cyclomethicone | 2,00 | 2,00 | 2,00 | 2,00 |
| Dicaprylyl Carbonate | - | 2,00 | 2,50 | 2,50 |
| Octyldodecanol | 1,0 | - | - | 1,50 |
| Butyrospermum Parkii Butter | 2,0 | - | - | - |
| Octyldodecyl Myristate | 1,0 | - | 1,5 | 1,0 |
| Dimethicone | 1,00 | 1,00 | 1,00 | 1,00 |
| Glycerin | 7,50 | 5,0 | 9,0 | 7,50 |
| Methylparaben | 0,20 | - | 0,1 | - |
| Phenoxyethanol | 0,40 | 0,50 | 0,40 | 0,40 |
| Propylparaben | 0,10 | - | - | - |
| Glyceryl Caprylate | - | 0,25 | - | - |
| Pentylenglykol | - | 0,5 | - | - |
| Butylenglykol | - | - | 3,0 | - |
| Carbomer | 0,15 | 0,10 | 0,10 | 0,15 |
| Sodium Polyacrylate | - | 0,20 | 0,20 | - |
| Xanthan Gummi | 0,10 | - | - | - |
| Acrylates/C10-30 Alkyl Acrylate Crosspolymer | - | - | - | 0,1 |
| Trisodium EDTA + Wasser (20%ige wässrige Lösung) | - | 1,00 | 1,00 | 1,00 |
| Tapioca Stärke | - | 1,00 | 1,00 | 1,00 |
| Distärke phosphat | - | 1,00 | 1,00 | 1,00 |
| Aluminum Stärke Octenylsuccinat | 2,0 | - | - | - |
| Acrylonitrile-methacrylonitrile-methyl-methacrylate Copolymer + Isopentane + Magnesium Hydroxide | 1,0 | - | - | - |
| Ethylhexyl Methoxycinnamat | - | 1,00 | 1,00 | 2,00 |
| Diethylamino Hydroxybenzoyl Hexyl Benzoat | - | 1,00 | - | 1,00 |
| Titaniumdioxid | - | - | 1,0 | - |
| Homosalate (3,3,5-Trimethyl-cyclohexylsalicylat) | - | - | 2,0 | - |
| Phenylbenzimidazole Sulfonic Acid | | | 1,0 | |
| Natrium Metabisulfite | 0,1 | - | - | - |
| Natriumhydroxid | q.s. | q.s. | q.s. | q.s. |
| Coffein | 0,01 | - | - | - |
| Tocopherol | - | 0,05 | - | - |
| (-)-Catechin | - | - | 0,1 | - |
| Ascorbylpalmitat | 0,1 | - | - | - |
| Parfüm | q.s. | q.s. | q.s. | q.s. |
| Wasser | ad 100 | ad 100 | ad 100 | ad 100 |

| **Rezepturbeispiele** | **17** | **18** | **19** | **20** |
|---|---|---|---|---|
| **INCI - Bezeichnung** | **Gew.-%** | **Gew.-%** | **Gew.-%** | **Gew.-%** |
| Glyceryl Stearate Citrat | 2,0 | 2,0 | 2,0 | 2,0 |
| Isopropyl Palmitat | 3,0 | 2,0 | 3,0 | 1,0 |
| Cetylstearylalkohol | 4,0 | 3,0 | 3,0 | - |
| Cetylalkohol | | | | 4,0 |
| Caprylsäure/Caprinsäure Triglyceride | 3,0 | 2,5 | 2,0 | 3,0 |
| C12-15 Alkyl Benzoat | 3,0 | 2,5 | 2,0 | 2,0 |
| Cyclomethicon | 1,0 | - | 1,0 | - |
| Dicaprylyl Carbonat | - | - | 2,5 | - |
| Dimethicon | - | 0,50 | - | - |
| Octyldodecyl Myristate | - | 1,0 | - | - |
| Glycerin | 4,00 | 6,00 | 5,00 | 6,00 |
| Methylparaben | 0,20 | - | 0,10 | - |
| Phenoxyethanol | 0,40 | 0,40 | 0,40 | 0,40 |
| Piroctone Olamine | - | - | - | 0,10 |
| Ethylhexylglycerin | - | 0,3 | - | - |
| Glyceryl Caprylate | - | 0,3 | - | - |
| 2-Methyl-1,3-propandiol | - | 2,0 | - | 2,0 |
| Carbomer | 0,20 | 0,10 | 0,15 | - |
| Natrium Polyacrylate | - | 0,40 | - | - |
| Xanthan Gummi | 0,10 | - | - | 0,15 |
| Acrylates/C10-30 Alkyl Acrylate Crosspolymer | - | - | 0,1 | 0,2 |
| Acrylonitrile-methacrylonitrile-methyl-methacrylate Copolymer + Isopentane + Magnesium Hydroxide | 0,50 | - | 0,50 | - |
| Aluminum Starch Octenylsuccinate | - | 1,00 | - | 1,00 |
| Methyl Methacrylate Crosspolymer | 1,0 | | | 1,0 |
| Bis-Ethylhexyloxyphenol Methoxyphenyl Triazines | - | | 1,0 | |
| Titandioxid | - | 1,00 | - | 1,00 |
| Octocrylene | - | 1,00 | 1,0 | 1,00 |
| Butyl Methoxydibenzoylmethane | - | 1,00 | - | 1,00 |
| Ethylhexyl Salicylate | - | - | 1,0 | - |
| Coffein | 0,05 | - | 0,005 | - |
| Tocopherol | 0,05 | 0,05 | - | 0,05 |
| (-)-Catechin | - | - | 0,05 | 0,05 |
| Natriumhydroxid | q.s. | q.s. | q.s. | q.s. |
| Wasser | ad 100 | ad 100 | ad 100 | ad 100 |

| **Rezepturbeispiele** | **21** | **22** | **23** | **24** |
|---|---|---|---|---|
| **INCI - Bezeichnung** | **Gew.-%** | **Gew.-%** | **Gew.-%** | **Gew.-%** |
| Sucrose Polystearate + Hydrogynated Polyisobutene | 1,00 | 1,00 | 2,00 | 2,00 |
| Sodium Stearoyl Glutamate | 0,20 | 0,20 | 0,30 | 0,30 |
| C12-15 Alkyl Benzoate | 1,50 | 1,50 | - | - |
| Cetyl Alcohol | 0,50 | 0,50 | - | - |
| Cyclomethicone | 10,00 | 10,00 | 5,00 | 5,00 |
| Dimethicone | 3,00 | 3,00 | 2,50 | 2,50 |
| Glycerin | 7,50 | 7,50 | 5,00 | 5,00 |
| Isopropyl Stearate | 1,00 | 1,00 | 2,00 | 2,00 |
| Paraffinum Liquidum | 3,00 | 3,00 | 1,00 | 1,00 |
| Methylparaben | 0,10 | - | - | 0,10 |
| Ethylhexylglycerin | - | - | 0,3 | 0,10 |
| Propylparaben | 0,1 | - | - | - |
| Methylisothiazolinon | - | 0,05 | - | - |
| Phenoxyethanol | 0,40 | 0,50 | 0,40 | 0,40 |
| Ethylhexyl Methoxycinnamate | 3,00 | 2,00 | 3,00 | 3,0 |
| Butyl Methoxydibenzoylmethane | 2,00 | 2,00 | 1,00 | 1,0 |
| Phenylbenzimidazole Sulfonic Acid | - | 1,5 | - | 1,0 |
| Butylenglykol | - | - | 3,0 | - |
| Polymethylsissesquioxane | - | - | 1,0 | 1,0 |
| Nylon-12 | - | 1,0 | 1,0 | - |
| Distärke Phosphat | - | 1,0 | - | 1,0 |
| Methyl Methacrylate Crosspolymer | 1,0 | - | - | - |
| Aluminum Starch Octenylsuccinat | 1,0 | - | - | - |
| Ammonium Acryloyldimethyltaurate/VP Copolymer | - | - | 0,25 | 0,25 |
| Xanthan Gummi | 0,10 | - | - | 0,1 |
| Acrylates/C10-30 Alkyl Acrylate Crosspolymer | 0,25 | 0,1 | - | - |
| Carbomer | - | 0,1 | 0,1 | - |
| Coffein | 0,005 | 0,01 | - | 0,1 |
| Tocopherol | - | 0,1 | 0,5 | - |
| (-)-Catechin | - | - | 0,05 | 0,1 |
| Parfüm | 0,2 | 0,2 | 0,2 | 0,2 |
| Natriumhydroxid | q.s. | q.s. | q.s. | q.s. |
| Wasser | ad 100 | ad 100 | ad 100 | ad 100 |

| **Rezepturbeispiele** | **25** | **26** | **27** | **28** |
|---|---|---|---|---|
| **INCI - Bezeichnung** | **Gew.-%** | **Gew.-%** | **Gew.-%** | **Gew.-%** |
| Natrium Cetearyl Sulfate | 0,15 | 0,15 | 0,15 | 0,15 |
| Glyceryl Stearate SE | 2,00 | 2,00 | 1,50 | 1,50 |
| C12-15 Alkyl Benzoate | 2,50 | 2,50 | 2,50 | 2,50 |
| Octyldodecanol | 1,00 | 1,00 | - | - |
| Caprylsäure/Caprinsäure Triglyceride | 2,00 | 2,00 | 2,00 | 2,00 |
| Cetylstearylalkohol | 2,00 | 2,00 | 3,00 | 1,00 |
| Cyclomethicone | 1,50 | 1,50 | 2,50 | 2,50 |
| Dimethicone | 0,50 | 0,50 | 0,50 | 0,50 |
| Glycerin | 5,00 | 5,00 | 7,50 | 7,50 |
| Isopropyl Stearat | 3,00 | 3,00 | 2,00 | 2,00 |
| Paraffinum Liquidum | 2,00 | 2,00 | 1,00 | 1,00 |
| Methylisothiazolinon | - | - | - | 0,05 |
| Phenoxyethanol | 0,4 | 0,5 | 0,40 | 0,30 |
| Methylparaben | 0,15 | - | - | - |
| Propylparaben | 0,1 | - | - | - |
| Piroctone Olamine | - | 0,15 | - | - |
| Benzethonium chlorid | - | - | 0,10 | - |
| Ethylhexyl Methoxycinnamate | 3,00 | 3,00 | 5,00 | 5,00 |
| Butyl Methoxydibenzoylmethane | 1,00 | 1,00 | 2,00 | 2,00 |
| Pentylenglykol | - | 1,0 | 1,0 | - |
| Butylenglycol | 1,0 | 1,5 | 3,0 | 3,0 |
| 2-Methyl-1,3-propandiol | - | - | - | - |
| 1,2-Hexandiol | - | - | - | 1,0 |
| Nylon-12 | 1,0 | 1,0 | 1,0 | 1,0 |
| Carbomer | - | - | 0,10 | 0,15 |
| Ammonium Acryloyldimethyltaurate/VP Copolymer | 0,20 | - | - | - |
| Chondrus Crispus | 0,10 | 0,10 | - | - |
| Xanthan Gummi | - | - | 0,10 | - |
| Acrylates/C10-30 Alkyl Acrylate Crosspolymer | - | 0,2 | 0,1 | 0,1 |
| Parfüm | 0,2 | 0,2 | 0,2 | 0,2 |
| Coffein | 0,05 | 0,05 | 0,05 | 0,1 |
| Tocopherol | - | 0,05 | 0,1 | - |
| (-)-Catechin | 0,2 | 0,05 | 0,1. | 0,05 |
| Natriumhydroxid | q.s. | q.s. | q.s. | q.s. |
| Wasser | ad 100 | ad 100 | ad 100 | ad 100 |

| **Rezepturbeispiele** | **29** | **30** | **31** | **32** |
|---|---|---|---|---|
| **Chemische Bezeichnung** | **Gew.-%** | **Gew.-%** | **Gew.-%** | **Gew.-%** |
| Natrium Cetearyl Sulfat | 0,15 | 0,15 | 0,2 | 0,2 |
| Glyceryl Stearate, selbstemulgierend | 2 | 2 | 1,5 | 1,5 |
| C12-15 Alkyl Benzoat | 2 | 2 | 2 | 2 |
| Octyldodecanol | 1 | 1 | - | - |
| Caprylsäure/Caprinsäure Triglyceride | 2 | 2 | 2 | 2 |
| Cetylstearylalkohol | 2 | 2 | 1 | 1 |
| Cyclomethicone | 1 | 1 | 2 | 2 |
| Dimethicone | 0,5 | 0,5 | 1 | 1 |
| Glycerin | 5 | 5 | 7,5 | 7,5 |
| Isopropyl Palmitat | 2,5 | 2,5 | 2 | 2 |
| DMDM Hydantoin | 0,05 | 0,05 | 0,05 | 0,05 |
| Phenoxyethanol | 0,35 | 0,25 | 0,3 | 0,3 |
| Ethanol | | | 3,0 | 2,0 |
| Pentylenglykol | 1,0 | | 1,0 | 1,5 |
| Carbomer | 0,2 | 0,2 | 0,2 | 0,2 |
| Carrageenan | 0,1 | 0,1 | - | - |
| Xanthan Gummi | - | - | 0,2 | 0,2 |
| Acrylates/C10-30 Alkyl Acrylate Crosspolymer | - | - | - | 0,15 |
| Natrium Polyacrylat | - | 0,2 | - | - |
| Diethylhexyl 2,6-Naphthalat | - | - | 1,0 | - |
| Phenylbenzimidazole Sulfonsäure | - | 1,0 | - | 2,0 |
| Titandioxid | - | - | 1,0 | - |
| Diethylamino Hydroxybenzoyl Hexyl Benzoate | - | - | 1,0 | 1,0 |
| Octocrylene | - | 4,0 | 2,0 | 3,0 |
| 3,3,5-Trimethylcyclohexylsalicylate | | 1,0 | - | - |
| Distärkepjosphat | - | 1,0 | 1,0 | - |
| Methyl Methacrylate Crosspolymer | 1,0 | - | - | 1,0 |
| Polymethylsissesquioxane | - | - | 1,0 | 1,0 |
| Acrylonitrile-methacrylonitrile-methyl-methacrylate Copolymer + Isopentane + Magnesium Hydroxide | 1,0 | 1,0 | - | - |
| Coffein | - | 0,1 | 0,1 | 0,5 |
| Tocopherol | 0,5 | 0,5 | 1,0 | 2,0 |
| (-)-Catechin | 0,05 | - | 0,005 | 0,05 |
| Parfüm | 0,15 | 0,15 | 0,3 | 0,3 |
| Natriumhydroxid | q.s. | q.s. | q.s. | q.s. |
| Wasser | ad 100 | ad 100 | ad 100 | ad 100 |

| **Rezepturbeispiele** | **33** | **34** | **35** | **36** |
|---|---|---|---|---|
| **INCI - Bezeichnung** | **Gew.-%** | **Gew**.**-%** | **Gew.-%** | **Gew.-%** |
| Natrium Cetearyl Sulfat | 0,15 | 0,15 | 0,15 | 0,15 |
| Glyceryl Stearate, selbstemulgierend | 1,0 | 1,0 | 1,0 | 1,0 |
| C12-15 Alkyl Benzoat | 2,0 | 2,5 | 2,0 | 2,0 |
| Isopropyl Palmitat | 3,5 | 3,0 | 2,5 | 3,5 |
| Dimethicon | 1,0 | 1,0 | 1,0 | 1,0 |
| Cetylstearylalkohol | 1,0 | 1,0 | 1,0 | 1,0 |
| Octyldodecyl Myristate | - | - | - | 1,0 |
| Butyrospermum Parkii Butter | - | - | 1,0 | - |
| Glycerin | 7,0 | 3,0 | 9,0 | 5,0 |
| Carbomer | 0,1 | 0,15 | 0,1 | 0,1 |
| Acrylates/C10-30 Alkyl Acrylate Crosspolymer | 0,15 | 0,1 | 0,1 | 0,15 |
| Xanthan Gummi | 0,15 | 0,15 | 0,15 | 0,15 |
| Phenylbenzimidazole Sulfonsäure | 1,0 | 1,0 | - | 1,0 |
| Butyl Methoxydibenzoylmethane | 1,5 | 1,5 | 1,5 | 1,5 |
| Ethylhexyl Salicylate | 2,0 | 2,5 | 2,5 | 2,5 |
| Octocrylene | 1,5 | 1,5 | 2,5 | 1,5 |
| TitanDioxide + Trimethoxycaprylylsilan | 1,0 | - | 1,0 | - |
| Aluminum Stärke Octenylsuccinat | - | 1,0 | - | 0,5 |
| Methyl Methacrylate Crosspolymer | 0,5 | - | 0,5 | - |
| Nylon-12 (Puderharz) | 0,5 | - | 1,0 | - |
| Tapioka Stärke | 0,5 | 0,5 | - | 1,0 |
| Phenoxyethanol | 0,5 | 0,5 | 0,5 | 0,4 |
| Ethylhexylglycerin | 0,25 | - | 0,25 | - |
| 1,2-Hexandiol | - | 1,0 | - | 3,0 |
| Caprylyl Glycol | - | 0,3 | 0,3 | - |
| 2-Methyl-1,2-propandiol | 2,0 | 2,0 | 2,0 | - |
| Coffein | 0,1 | - | 0,1 | - |
| Tocopherol | - | 2,0 | - | 3,0 |
| (-)-Catechin | 0,05 | - | 0,05 | - |
| Natriumhydroxid | q.s. | q.s. | q.s. | q.s. |
| Wasser | ad 100 | ad 100 | ad 100 | ad 100 |

| **Rezepturbeispiele** | **37** | **38** | **39** | **40** |
|---|---|---|---|---|
| **INCI - Bezeichnung** | **Gew.-%** | **Gew.-%** | **Gew.-%** | **Gew.-%** |
| Polyglycerly-10 Stearat | 0,2 | 0,20 | 0,20 | 0,20 |
| Glyceryl Stearat | 3,0 | 0,50 | 0,50 | 0,50 |
| C12-15 Alkyl Benzoat | 4,0 | 2,0 | 1,5 | 2,5 |
| Isopropyl Palmitat | 4,0 | 1,0 | 2,0 | 2,5 |
| Caprylsäure/Caprinsäure Triglyceride | 4,0 | 3,0 | 2,0 | 2,5 |
| Hydrogenated Coco-Glycerides | 3,0 | - | - | 2,0 |
| Butyrospermum Parkii Butter | 3,0 | - | 2,5 | - |
| Cetylstearylalkohol | 5,0 | 3,5 | 4,0 | 3,0 |
| Paraffinum Liquidum | - | - | - | 1,0 |
| Glycerin | 5,0 | 3,0 | 7,0 | 9,0 |
| Acrylates/C10-30 Alkyl Acrylate Crosspolymer | 0,3 | 0,2 | 0,15 | 0,2 |
| Methylisothiazolinone | 0,05 | - | - | 0,05 |
| Phenoxyethanol | 0,5 | 0,4 | 0,4 | 0,4 |
| Carbomer | 0,1 | 0,15 | 0,1 | 0,1 |
| Methylparaben | - | 0,1 | 0,1 | - |
| Propylparaben | - | 0,1 | - | - |
| Nylon-12 (Puderharz) | 1,0 | 0,5 | - | - |
| Polymethylsissesquioxane | - | 1,0 | 0,5 | - |
| Methyl Methacrylate Crosspolymer | - | - | 1,0 | 0,5 |
| Tapioka Stärke | 0,5 | - | - | 0,5 |
| Ethanol | 3,0 | - | 2,0 | - |
| Hexylsalicylat | 0,05 | 0,005 | - | - |
| Coffein | - | 0,5 | 0,5 | - |
| Tocopherol | - | - | 0,5 | 0,5 |
| (-)-Catechin | 0,05 | | | 0,05 |
| Parfüm | 0,2 | 0,15 | 0,3 | 0,3 |
| Natriumhydroxid | q.s. | q.s. | q.s. | q.s. |
| Wasser | ad 100 | ad 100 | ad 100 | ad 100 |

| **Rezepturbeispiele** | **41** | **42** | **43** | **44** |
|---|---|---|---|---|
| **INCI - Bezeichnung** | **Gew.-%** | **Gew.-%** | **Gew.-%** | **Gew.-%** |
| Polyglycerly-10 Stearat | 0,2 | 0,2 | 0,15 | 0,15 |
| C12-15 Alkyl Benzoat | 2,5 | 2,5 | 2,0 | 3,0 |
| Isopropyl Palmitat | 2,5 | 2,5 | 2,0 | 2,0 |
| Caprylsäure/Caprinsäure Triglyceride | 2,0 | 2,5 | 1,0 | 2,0 |
| Glyceryl Stearat | 1,0 | 1,0 | 0,5 | 0,5 |
| Octyldodecanol | 0,5 | - | - | 1,0 |
| Cyclomethicon | - | - | 0,5 | 0,5 |
| Butyl Methoxydibenzoylmethane | - | 2,0 | 2,0 | - |
| Octocrylene | - | 2,0 | 3,0 | 2,0 |
| Ethylhexyl Salicylate | - | 1,0 | 1,0 | - |
| Phenylbenzimidazole Sulfonic Acid | - | 1,0 | - | 1,5 |
| Titandioxid | - | 1,0 | - | 1,0 |
| 3,3,5-Trimethylcyclohexylsalicylate | - | - | 1,0 | 1,0 |
| Glycerin | 9,0 | 5,0 | 7,0 | 7,0 |
| Tapioka Stärke | 1,0 | 1,0 | - | - |
| Acrylonitrile-methacrylonitrile-methyl-methacry-late Copolymer + Isopentane + Magnesium Hydroxide | - | 1,0 | 0,5 | - |
| Aluminum Stärke Octenylsuccinat | - | - | 1,0 | 1,0 |
| Distärkephosphat | - | - | - | 1,0 |
| Methylisothiazolinone | 0,05 | 0,05 | - | - |
| Phenoxyethanol | 0,5 | 0,5 | 0,4 | 0,4 |
| Benzethoniumchlorid | - | - | 0,1 | - |
| Ethylhexylglycerin | - | - | 0,1 | - |
| Methylparaben | - | - | - | 0,2 |
| Carbomer | 0,25 | 0,2 | 0,2 | 0,2 |
| Acrylates/C10-30 Alkyl Acrylate Crosspolymer | 0,2 | - | - | 0,15 |
| Ammonium Acryloyldimethyltaurate/VP Copolymer | - | 0,25 | - | - |
| Natrium Polyacrylat | - | - | 0,3 | - |
| Xanthan Gummi | - | - | - | 0,15 |
| Ethanol | 3,0 | 3,0 | - | - |
| Butylenglykol | - | - | 2,0 | 2,0 |
| Cumarin | - | 0,05 | 0,05 | - |
| Coffein | 0,05 | 0,05 | - | 0,1 |
| Tocopherol | - | - | 1,0 | 0,5 |
| (-)-Catechin | 0,05 | 0.05 | | |
| Parfüm | 0,15 | 0,2 | 0,25 | 0,3 |
| Natriumhydroxid | q.s. | q.s. | q.s. | q.s. |
| Wasser | ad 100 | ad 100 | ad 100 | ad 100 |

| **Rezepturbeispiele** | **45** | **46** | **47** | **48** |
|---|---|---|---|---|
| **INCI - Bezeichnung** | **Gew.-%** | **Gew.-%** | **Gew.-%** | **Gew.-%** |
| Kalium Cetylphosphat | 0,20 | 0,20 | 0,25 | 0,20 |
| C12-15 Alkyl Benzoat | 2,5 | 2,5 | 2,0 | 2,0 |
| Isopropyl Palmitat | 2,5 | 2,5 | - | 3,0 |
| Isopropy Stearat | - | - | 2,0 | - |
| Caprylsäure/Caprinsäure Triglyceride | 2,5 | 2,5 | 1,5 | 2,0 |
| Glyceryl Stearate | 1,0 | 1,0 | 1,25 | 1,5 |
| Octyldodecanol | - | - | 1,5 | - |
| Paraffinum Liquidum | - | - | - | 1,0 |
| Glycerin | 5,0 | 7,0 | 9,0 | 6,0 |
| Bis-Ethylhexyloxyphenol Methoxyphenyl Triazines | - | 1,0 | - | 1,0 |
| TitanDioxide + Trimethoxycaprylylsilane | - | - | 1,0 | 1,0 |
| Phenylbenzimidazole Sulfonic Acid | - | - | 1,0 | 1,0 |
| Butyl Methoxydibenzoylmethane | 1,0 | - | 2,0 | 2,0 |
| Dinatrium Phenyl Dibenzimidazol Tetrasulfonat | - | 1,5 | 1,0 | - |
| Ethylhexyltriazon | 1,0 | - | - | - |
| Ethylhexyl Methoxycinnamat + BHT | 2,0 | - | - | - |
| Carbomer | | 0,15 | 0,2 | 0,3 |
| Acrylates/C10-30 Alkyl Acrylate Crosspolymer | 0,3 | 0,1 | 0,15 | - |
| Xanthan Gummi | | | 0,15 | 0,1 |
| Methylisothiazolinone | 0,05 | - | - | - |
| Phenoxyethanol | 0,5 | 0,5 | 0,4 | 0,4 |
| Methylparaben | - | 0,1 | - | - |
| Ethylhexylsalicylat | - | - | 0,3 | - |
| Butylenglycol | - | - | 3,0 | 3,0 |
| Benzethonium chlorid | - | - | - | 0,1 |
| Coffein | 0,05 | - | - | 0,1 |
| Tocopherol | 0,05 | 0,5 | - | - |
| (-)-Catechin | - | - | 0,1 | - |
| Parfüm | 0,1 | 0,3. | 0,2 | 0,3 |
| BHT (tert-Butylhydroxytoluol) | 0,05 | - | - | - |
| Tocopheryl Acetat | - | 0,1 | - | - |
| Natriumhydroxid | q.s. | q.s. | q.s. | q.s. |
| Wasser | ad 100 | ad 100 | ad 100 | ad 100 |

| **Rezepturbeispiele** | **49** | **50** |
|---|---|---|
| **INCI - Bezeichnung** | **Gew.-%** | **Gew.-%** |
| Polygylceryl-3 Diisostearate | 1,5 | 1,5 |
| PEG-40 Sorbitan Perisostearate | 2,5 | 2,5 |
| Lanolin Alkohol | 0,5 | 0,5 |
| Mineralöl | 8 | 8 |
| Cera Microcrystallina | 2,5 | 2,5 |
| Cyclomethicone | 4 | 4 |
| Isohexadecan | 2 | 2 |
| Isopropylpalmitat | 5 | 5 |
| lodopropynyl Butylcarbamate | - | 0,1 |
| Magnesiumsulfat | 0,5 | 0,5 |
| Potassium Sorbate | 0,1 | - |
| Tocopherol | 0,1 | - |
| Phenylethylalkohol | - | 0,1 |
| Glycerin | 7 | 7 |
| Linalool | 0,005 | 0,05 |
| Wasser | ad 100 | ad 100 |

## Patentansprüche

1. Verwendung von einer oder mehreren Substanz(en), gewählt aus folgender Gruppe mit anellierten / kondensierten bizyklischen Verbindungen
Coffein (58-08-2), Tocopherol (10191-41-0) und (-)-Catechin (18829-70-4)
zur Stimulierung der Expression von Endo180 zur Aktivierung des Kollagen-Stoffwechsels in der Altershaut.

2. Verwendung von einer oder mehreren Substanz(en), gewählt aus folgender Gruppe mit anellierten / kondensierten bizyklischen Verbindungen
Coffein (58-08-2), Tocopherol (10191-41-0) und (-)-Catechin (18829-70-4)
zur Stimulierung der Expression von Endo180 zur Anregung der Altershaut, defektes Kollagen zu beseitigen.

3. Verwendung nach einem der vorstehenden Ansprüche, **dadurch gekennzeichnet, dass** eine oder mehrere Substanz(en) aus der Gruppe Tocopherol (10191-41-0) und (-)-Catechin (18829-70-4) gewählt werden.

4. Verwendung nach einem der vorstehenden Ansprüche, **dadurch gekennzeichnet, dass** die Substanz(en) mit einem Gehalt von 0,00001 - 50,0 Gew.-%, insbesondere 0,001 - 20,0 Gew.-%, bezogen auf das Gesamtgewicht der Zusammensetzung in kosmetischen oder dermatologischen Zusammensetzungen eingesetzt werden.

5. Verwendung nach Anspruch 4 **dadurch gekennzeichnet, dass** die Substanz(en) mit 0,002 - 10,0 Gew.-%, besonders bevorzugt 0,002 - 5,0 Gew.-%, ganz besonders bevorzugt 0,005 - 3,0 Gew.-%, jeweils bezogen auf das Gesamtgewicht der Zusammensetzung in kosmetischen oder dermatologischen Zusammensetzungen eingesetzt werden.
